Europäisches Patentamt

European Patent Office

Office européen des brevets

(11) Numéro de publication : **0 334 756 B1**

(12) **FASCICULE DE BREVET EUROPEEN**

(45) Date de publication du fascicule du brevet :
**24.06.92 Bulletin 92/26**

(51) Int. Cl.$^5$ : **C12Q 1/68,** C12Q 1/42,
C12Q 1/26

(21) Numéro de dépôt : **89400801.0**

(22) Date de dépôt : **21.03.89**

(54) **Procédé de coloration d'acides nucléiques détectés par marquage non-radioactif et ensemble de réactifs pour la mise en oeuvrede ce procédé.**

(30) Priorité : **25.03.88 FR 8803982**
**10.03.89 FR 8903191**

(43) Date de publication de la demande :
**27.09.89 Bulletin 89/39**

(45) Mention de la délivrance du brevet :
**24.06.92 Bulletin 92/26**

(84) Etats contractants désignés :
**AT BE CH DE ES FR GB GR IT LI LU NL SE**

(56) Documents cités :
**CHEMICAL ABSTRACTS, vol.92, 1980, Columbus, OH (US); J.MARCANIK et al., p.75, no.15690y**
**CHEMICAL ABSTRACTS, vol.97, no.9, 30 août 1982, Columbus, OH (US); G.W.RAFTER, p.38, no.66124c**

(73) Titulaire : **BIOPROBE SYSTEMS**
**33, rue de la Brèche aux Loups**
**F-75012 Paris (FR)**

(72) Inventeur : **Lebacq, Philippe**
**33, rue de la Brèche aux Loups**
**F-75012 Paris (FR)**

(74) Mandataire : **Netter, André et al**
**Cabinet NETTER, 40, rue Vignon**
**F-75009 Paris (FR)**

EP 0 334 756 B1

Jouve, 18, rue Saint-Denis, 75001 PARIS

## Description

L'invention concerne la coloration d'acides nucléiques, préalablement fixés sur un support solide, détectés par un procédé de marquage de type non-radioactif.

La détection de séquences d'acides nucléiques spécifiques aux êtres biologiques revêt une importance de plus en plus grande, notamment pour la mise en évidence de maladies génétiques.

On connaît déjà différents procédés qui utilisent un support solide, par exemple une membrane en polyamide ou en nitrocellulose, sur lequel on transfère et fixe les acides nucléiques d'un échantillon à analyser, et on détecte ensuite sur le support la présence éventuelle de la séquence recherchée.

L'acide nucléique d'un échantillon peut être un ADN ou un ARN, naturel ou synthétique, modifié ou non. Cet acide nucléique est habituellement découpé en fragments au moyen d'enzymes de restriction et ces fragments sont ensuite transférés et fixés sur le support par différents moyens en eux-mêmes connus.

La présence éventuelle de la séquence recherchée est alors mise en évidence par un système de détection, tel qu'une "sonde", susceptible de se fixer spécifiquement sur la séquence et de la révéler ainsi directement in situ, c'est-à-dire sur le lieu même de sa fixation sur le support solide.

Généralement, les systèmes de détection utilisés comprennent une séquence complémentaire de la séquence à identifier et susceptible de s'hybrider avec celle-ci. Une telle hybridation n'est alors obtenue que dans le cas où le système de détection reconnaît une séquence complémentaire fixée sur le support solide.

Il est possible également, dans certains procédés, d'obtenir une détection directe des acides nucléiques, et cela sans faire appel à une hybridation entre une séquence "cible" portée par le support solide et une séquence "sonde" faisant partie du système de détection.

Une telle détection d'acides nucléiques, que ce soit directement ou après hybridation entre deux séquences d'acides nucléiques complémentaires, a été effectuée pendant de nombreuses années à l'aide d'isotopes radioactifs. Dans les procédés connus de ce type, on utilise des nucléosides ou des nucléotides marqués radioactivement au 3-H ou au 32-P, afin de marquer les acides nucléiques et les détecter par comptage ou par autoradiographie. Si ces procédés connus donnent entière satisfaction en ce qui concerne la fiabilité et la sensibilité de la détection, ils ont malheureusement pour inconvénient de nécessiter des laboratoires spécialement équipés pour la radioactivité. En outre, ces laboratoires doivent avoir reçu les autorisations nécessaires pour manipuler, utiliser et confiner des isotopes radioactifs.

Pour éviter pareil inconvénient, différents procédés de détection n'utilisant pas d'isotopes radioactifs ont été développés au cours des dernières années.

C'est ainsi que l'on connaît des procédés de détection d'acides nucléiques préalablement fixés sur un support solide tel qu'une membrane, dans lesquels on utilise un système enzymatique propre à se fixer sur cette séquence par un marquage de type non radioactif, et dans lesquels on fait ensuite réagir ce système enzymatique sur un substrat chromogénique pour former un produit coloré qui précipite in situ, ce qui permet de révéler la séquence détectée sur le site même de la réaction.

Un tel procédé de détection est mis en oeuvre par un ensemble de réactifs, encore appelé "kit", qui contient les produits nécessaires à la caractérisation des acides nucléiques à rechercher.

La plupart des "kits" de détection des acides nucléiques, que l'on trouve dans le commerce, utilisent comme enzyme finale de visualisation la phosphatase alcaline. Cette enzyme possède, en effet, des qualités telles que les procédés de détection non-radioactifs utilisant cette enzyme sont tout aussi performants que les procédés de détection radioactifs utilisant des isotopes.

Il est à noter que la phosphatase alcaline est une enzyme particulièrement résistante qui donne un niveau de sensibilité maximale par rapport aux autres enzymes utilisées dans ce type de détection, comme la peroxydase ou la glucosidase.

L'avantage essentiel des procédés et systèmes non-radioactifs se situe avant tout dans leur utilisation dans le domaine diagnostique, dans un environnement où l'usage de la radioactivité trouve difficilement sa place pour constituer des tests de routine. Ces nouveaux tests diagnostiques destinés à remplacer certains tests connus, en particulier les tests dits "ELISA" touchent tous les domaines, notamment la bactériologie, la virologie et la parasitologie. Ils sont pratiquement utilisables partout où se trouvent des acides nucléiques détectables, c'est-à-dire chez l'ensemble des organismes vivants.

L'utilisation de "sondes" non-radioactives dites "froides" présente également un progrès notable en recherche fondamentale et en recherche clinique, dès lors que la sensibilité de détection est au moins égale à celle obtenue avec des isotopes radioactifs.

En effet, les "sondes" non-radioactives ont pour avantage essentiel d'être d'un emploi aisé et de ne pas nécessiter d'équipements spéciaux. Par ailleurs, elles ne présentent pas les risques pour l'utilisateur, liés à l'utilisation de radio-isotopes et sont de plus sans danger dans la mesure où elles ne font pas appel à des produits toxiques ou carcinogènes.

Cependant, si elles présentent de nombreux avantages par rapport aux sondes radioactives, les "sondes froides" présentent aussi certains inconvénients.

Certains d'entre eux ont déjà pu être levés, comme c'est le cas pour la détection désormais possible de "séquences uniques".

Un des principaux inconvénients des "sondes froides" réside dans l'impossibilité de réutiliser les supports solides, par exemple les membranes, après hybridation et détection colorimétrique. Cet inconvénient pose un problème majeur en recherche clinique et fondamentale.

En effet, les chercheurs doivent réutiliser les mêmes supports sur lesquels sont fixés irréversiblement les acides nucléiques-cibles, et cela de nombreuses fois, par exemple jusqu'à une vingtaine de fois dans certains cas.

Les acides nucléiques fixés peuvent être précieux, soit du fait que leur extraction ait été longue, fastidieuse et coûteuse, soit encore que ces acides nucléiques soient rares, comme c'est le cas de l'ADN foetal par exemple. Il est donc important de pouvoir fixer de nombreuses fois, par exemple par réhybridation, des sondes sur les acides nucléiques-cibles considérés.

Une telle réhybridation est parfaitement courante pour les sondes isotopiques, mais encore impossible avec les "sondes froides" du fait du dépôt indélébile constitué par le produit coloré de visualisation qui précipite in situ.

Différentes tentatives ont été menées dans le but de pouvoir enlever le produit coloré formé, mais ces tentatives n'ont jusqu'à présent pas abouti. Ainsi, certains fabricants ont préconisé d'utiliser le diméthylformamide à chaud pour éliminer cette coloration indélébile, mais malheureusement le diméthylformamide est un agent tératogène volatile, notamment à chaud, ce qui en limite l'usage en bains répétés.

Par conséquent, il n'existe pas à l'heure actuelle de procédé de révélation colorimétrique utilisant des substrats qui donnent des produits colorés insolubles que l'on puisse éliminer facilement après visualisation dans le but de réutiliser les supports solides sur lesquels ont été transférés et fixés, de façon irréversible, les acides nucléiques à analyser.

C'est, en conséquence, un but de l'invention de procurer un procédé de coloration d'acides nucléiques, préalablement fixés sur un support solide approprié, qui fait appel à une détection par un marquage de type non-radioactif et qui permet d'éliminer le produit coloré de visualisation de la réaction.

C'est également un but de l'invention de procurer un tel procédé qui permet d'éliminer ce produit coloré en faisant appel à des produits qui ne présentent aucun danger pour l'utilisateur.

C'est également un but de l'invention de procurer un tel procédé qui permet aussi une bonne visualisation et donc une bonne détection des acides nucléiques, notamment la visualisation de séquences génomiques uniques.

C'est aussi un but de l'invention de procurer un tel procédé qui puisse être appliqué en particulier sur des supports solides constitués par des membranes en polyamide ou en nitrocellulose, sur lesquelles sont fixés irréversiblement les acides nucléiques-cibles à analyser.

C'est aussi un but de l'invention de procurer un tel procédé qui permet une décoloration facile du support solide utilisé, notamment dans le cas d'une membrane, de manière à obtenir un support parfaitement blanc, sans trace de coloration, qui peut être réutilisé immédiatement pour une nouvelle opération de détection , par exemple par hybridation.

C'est enfin un but de l'invention de procurer un ensemble de réactifs ou "kit" pour la mise en oeuvre d'un tel procédé de détection.

L'invention concerne plus particulièrement un procédé de coloration d'une séquence d'acide nucléique préalablement fixée sur un support solide, dans lequel on utilise un système enzymatique propre à se fixer sur cette séquence pour la détecter par un marquage de type non-radioactif, et dans lequel on fait réagir ce système enzymatique sur un substrat chromogénique pour former un produit coloré qui précipite in situ, ce qui permet de révéler la séquence détectée directement sur le site même de la réaction.

Conformément à la caractéristique essentielle de l'invention, on utilise une solution de révélation comprenant :

– un composé organique soufré, possédant une liaison avec un atome de soufre, formant substrat pour le système enzymatique, et propre à être hydrolysé par le système enzymatique pour former un composé organique à fonction thiol; et

– un composé de métal, soluble et stable en solution aqueuse, propre à réagir avec le composé organique à fonction thiol pour former un complexe métal-composé soufré, qui présente une coloration spécifique dudit complexe, qui précipite in situ et qui peut être ensuite décoloré.

Le métal du composé de métal est choisi avantageusement parmi l'or, le mercure, l'argent, le platine et le plomb.

De préférence, on utilise un composé de l'or qui est propre à réagir avec le composé organique à fonction thiol pour former un complexe or-composé soufré de coloration jaune.

Dans une forme de réalisation préférée de l'invention, on fait ensuite réagir le complexe métal-composé soufré ainsi obtenu, avec un composé renforçateur propre à accentuer la coloration dudit complexe. En effet, la coloration du complexe obtenu, même si elle est parfaitement détectable visuellement, ne se prête pas toujours bien à des clichés photographiques. Ceci est le cas en particulier de la coloration jaune, spécifique du complexe or-composé soufré.

Selon une autre caractéristique de l'invention, le complexe métal-composé soufré ainsi obtenu, dont la coloration peut être éventuellement renforcée, est traité ensuite par une solution de décoloration, ce qui permet de réutiliser le support sur lequel a été fixé au préalable, et de façon irréversible, l'acide nucléique à analyser.

Comme indiqué plus haut, le procédé de l'invention s'applique à la détection d'ADN ou ARN, naturels ou synthétiques, modifiés ou non qui ont été préalablement fixés de façon irréversible sur un support solide approprié.

Ces supports solides sont bien connus dans cette technique et il peut s'agir de support en matière plastique, par exemple en polystyrène, en latex, etc...

Toutefois, dans une forme de réalisation préférée de l'invention, le procédé est mis en oeuvre avec des membranes en polyamide (nylon) ou en nitrocellulose.

Les acides nucléiques provenant de l'échantillon à analyser sont de préférence soumis à un traitement par des enzymes de restriction pour les fractionner et sont ensuite transférés sur le support solide, conformément à l'une quelconque des techniques connues à cet effet.

Par exemple, l'acide nucléique, préalablement fragmenté est disposé dans un récipient contenant un gel d'agarose lequel est ensuite transféré par capillarité sur le support. En variante, on peut aussi effectuer un dépôt direct de l'acide nucléique en se servant du support comme filtre.

Dans tous les cas, l'acide nucléique transféré sur le support, en particulier sur la membrane, est fixé de façon irréversible sur ce support par séchage, par traitement aux ultra-violets, ou par un traitement de cuisson, ainsi qu'il est bien connu dans cette technique.

Dans la phase ultérieure du procédé de l'invention, le support solide sur lequel est fixé l'acide nucléique est ensuite traité par un système enzymatique approprié qui peut comporter ou non une séquence d'acide nucléique "sonde" complémentaire de la séquence d'acide nucléique "cible" fixé sur le support solide. Cette enzyme doit donc être capable de se fixer directement sur l'acide nucléique "sonde" ou sur l'acide nucléique "cible", ou encore indirectement par le biais d'un anticorps conjugué, d'une protéine ou de tout autre composé chimique.

Dans une forme de réalisation préférée de l'invention, le système enzymatique utilisé comprend une enzyme à activité phosphatasique, par exemple la phosphatase alcaline.

Dans ce cas, la solution de révélation utilisée doit comprendre un substrat approprié, comme par exemple la cystéamine-S-phosphate.

Comme indiqué plus haut, le composé organique soufré, qui forme substrat pour le système enzymatique, est propre à être hydrolysé par l'enzyme pour former un composé organique à fonction thiol. Dans le cas particulier où l'on utilise un système enzymatique à base de phosphatase alcaline, le substrat de l'enzyme est un composé possédant une liaison thio-ester avec un groupe phosphate reconnu par l'enzyme. L'hydrolyse de cette liaison par l'enzyme libère un ion phosphate et un composé organique doté d'une fonction thiol, c'est-à-dire un composé de type R-SH (où R est radical organique) capable de réagir avec certains composés de métal, qui sont solubles et stables en solution aqueuse, pour former ainsi un complexe métal-composé soufré, de coloration spécifique.

Au lieu d'utiliser un système enzymatique comprenant une enzyme phosphatasique, on peut utiliser un système comprenant l'enzyme glutathion réductase et utiliser alors un substrat de cette enzyme particulière.

Le composé de métal à utiliser dans le procédé de l'invention doit être soluble et stable en solution aqueuse et capable de réagir avec le composé organique à fonction thiol, indiqué plus haut, pour former un complexe métal-composé soufré, qui présente une coloration spécifique dudit complexe et qui précipite in situ.

Dans la forme de réalisation préférée où le composé de métal est un composé de l'or, il peut s'agir, par exemple, d'un composé organique de l'or ou encore d'un sel d'or.

C'est ainsi que l'on peut utiliser un composé de l'or dans lequel cet or est présent sous la forme Au (I), qui correspond au degré le plus commun d'oxydation de l'or. Sous cette forme l'or donne un complexe insoluble avec la L-cystéine dans l'eau. Cette réaction se fait mole à mole et donne un complexe de nature polymérique, la fixation de l'or se faisant au niveau de l'atome de soufre de la cystéine.

Il existe en fait peu de composés qui stabilisent l'or sous la forme Au (I) en solution aqueuse. Parmi ces composés on peut citer essentiellement l'aurothiomalate de sodium (Myocrisine) et l'aurothioglucose (Solganol).

Dans une forme de réalisation préférée de l'invention, on utilise l'aurothioglucose car il s'agit d'un composé très stable en solution aqueuse. Il réagit avec la fonction thiol du produit d'hydrolyse de la réaction enzymatique pour former un complexe de coloration jaune citron à jaune d'or qui précipite in situ sur le lieu même de la réaction.

Le composé de l'or, tel qu'utilisé dans la solution de révélation de l'invention, peut être également constitué par un sel d'or, également soluble et stable en solution aqueuse, comme par exemple $HAuCl_4$.

La solution de révélation utilisée pour la mise en oeuvre du procédé de l'invention est de préférence mélangée à un tampon approprié à pH neutre ou légèrement basique. A titre d'exemple, ce tampon peut être à base de Tris-acétate et d'acétate de magnésium.

La solution de révélation est à préparer de façon extemporanée, préalablement à l'utilisation, en mélangeant ensemble le tampon, le composé organique soufré formant substrat et le composé de métal, par exemple le composé de l'or.

On obtient ainsi une solution aqueuse qui peut être utilisée directement pour la révélation de l'acide nucléique recherché.

La coloration spécifique du complexe métal-composé soufré peut être accentuée par traitement avec un composé renforçateur. Ce composé est de préférence un sel d'argent, par exemple du nitrate d'argent ou du perchlorate d'argent, propre à réagir sur ce complexe pour donner un complexe de coloration brun-noir. Une telle coloration est parfaitement visible et se prête particulièrement à des prises de clichés photographiques.

Grâce au procédé de l'invention, la coloration du précipité obtenu, avec ou sans addition du composé renforçateur, peut être éliminée par traitement avec une solution de décoloration.

Conformément à l'invention, cette solution est constituée avantageusement par un mélange de thiosulfate de sodium et de thiosulfate d'ammonium à forte molarité en milieu acide.

Après traitement par la solution de décoloration, le support peut être traité par un mélange d'urée et de SDS (Sodium Dodécyl Sulfate) à chaud pour éliminer toute trace de protéines et d'acides nucléiques, autre que la séquence d'acide nucléique restant fixée sur le support solide.

Pour la mise en oeuvre du procédé de l'invention, on peut prévoir un ensemble de réactifs ou "kit" comprenant les réactifs essentiels pour ce procédé.

De préférence, cet ensemble de réactifs comprendra au minimum un flacon de substrat, un flacon du composé de métal, un flacon d'une solution tampon propre à être mélangée au substrat et au composé de métal pour former la solution de révélation de façon extemporanée, et enfin un flacon d'une solution de décoloration.

De préférence, cet ensemble de réactifs comprendra en outre un flacon d'une solution d'un composé renforçateur de coloration et une solution d'un mélange d'urée et de SDS.

L'invention sera maintenant expliquée plus en détail à l'aide des exemples suivants :

- Exemple 1

dans cet exemple, on utilise un système enzymatique comprenant de la phosphatase alcaline.

On prépare, de façon extemporanée, une solution de révélation à partir des réactifs suivants :
– Tris-acétate 50 mM pH : 9,5,
– acétate de magnésium 10mM,
– cystéamine-phosphate à 5 mM,
– aurothioglucose à 3 mM.
On utilise cette solution de révélation sur un support solide, par exemple une membrane, sur laquelle a été fixé au préalable l'acide nucléique à analyser.

La révélation se développe en quinze minutes à deux heures selon le type d'hybridation ou de détection.

La cystéamine-phosphate est un composé de formule $NH_2$-$CH_2$-$CH_2$-S-$PO_3H_2$ qui libère la cystéamine de formule $NH_2$-$CH_2$-$CH_2$-SH après hydrolyse par la phosphatase alcaline. L'aurothioglucose réagit avec la fonction thiol de la cystéamine formée par l'hydrolyse enzymatique de la cystéamine-phosphate et donne ainsi un complexe de coloration jaune citron à à jaune d'or qui précipite in situ au lieu même de la réaction.

La visualisation des acides nucléiques modifiés ou des hybrides nucléiques est accentuée par une réaction entre le complexe aurothioglucose-cystéamine et du perchlorate ou du nitrate d'argent qui donne une coloration brun-marron à noir parfaitement visible.

Dans l'exemple, on utilise le perchlorate ou le nitrate d'argent en solution à 100 mM. Dès que l'apparition du complexe aurothioglucose-cystéamine a été jugée suffisante, la membrane est rincée brièvement dans l'eau distillée et on ajoute la solution de sels d'argent.

La réaction argent-complexe aurothioglucose-cystéamine est instantanée ou quasi instantanée. Après apparition de la coloration brune, la membrane est rincée à l'eau distillée et l'on peut prendre un cliché photographique des résultats.

L'apparition de cette coloration est spécifique du lieu où la phosphatase alcaline a été fixée, que cette fixation soit directe sur l'acide nucléique (ADN ou ARN) sonde, sur l'acide nucléique (ARN ou ADN) cible, ou indirecte par le biais d'un anticorps conjugué, d'une protéine ou de tout autre composé chimique.

La sensibilité du procédé est égale à celle obtenue avec les colorants classiques utilisés dans la révélation d'hybrides nucléiques non-radioactifs. Ces colorants peuvent être un système 5-Bromo-4-Chloro-3-Indolyle-phosphate (BCIP) et nitrobleu de tétrazolium (NBT).

Les résultats ainsi obtenus montrent en outre un abaissement significatif du bruit de fond et une meilleure définition des bandes détectées.

Grâce au procédé ci-dessus, il a été possible de détecter en hybridation dite "dot blot" jusqu'à 1 pg d'ADN et visualiser l'hybridation de séquences génomiques uniques après transfert de type "Southern" sur membrane. Il s'agit d'une empreinte sur membrane de nitrocellulose ou de polyamide d'une électrophorèse sur gel d'agarose d'un acide nucléique hydrolysé par une enzyme de restriction.

La couleur spécifique apparue sur les membranes considérées n'est pas indélébile et peut être ôtée après visualisation et prise de cliché.

Le traitement des membranes pour la décoloration est effectué en utilisant une solution de décoloration comprenant du thiosulfate d'ammonium 1M, du thiosulfate de sodium 1M, de l'acétate de sodium 0,1 M pH 4,8. En variante, on pourrait utiliser, pour la décoloration, du fixateur rapide pour film photographique, dilué quatre fois dans une solution de thiosulfate de sodium à 2 M. A titre d'exemple, un tel fixateur peut provenir de la firme GUILLEMINOT.

La décoloration est effectuée en plaçant la membrane immergée dans la solution de décoloration pendant environ quinze minutes à la température de la pièce.

La membrane est ensuite placée dans un sac en matière plastique et on ajoute une solution d'urée 7 M, 1% SDS à raison de 100 μl/cm². Le sac est scellé et immergé quinze à trente minutes dans un bain-marie à 70°C. La membrane est ensuite rincée à l'eau distillée à température ambiante, séchée et stockée entre deux papiers de type whatman, en vue d'une opération de détection ultérieure. Le stockage peut se faire à la température ambiante.

- Exemple 2

on utilise un système enzymatique comprenant l'enzyme glutathion réductase.

On utilise ce système enzymatique pour traiter une membrane sur laquelle a été transféré et fixé au préalable l'acide nucléique à analyser.

On prépare, de façon extemporanée, une solution de révélation à partir des réactifs suivants :
– Tris-HCl 50 mM à pH 7,
– NADPH* 5 mM,
– Glutathion sous forme oxydée (sels de sodium) 5 mM,
– HAuCl$_4$ ou Aurothioglucose 5 mM.

Les phases ultérieures du procédé se déroulent de la même façon que celles du procédé de l'exemple 1.

Le procédé de l'invention trouve ainsi une application dans de nombreux domaines, notamment en bactériologie, virologie et parasitologie, en améliorant très sensiblement les performances d'utilisation des "sondes froides" connues.

Ces applications couvrent en particulier la détection de maladies génétiques chez le foetus à partir de prélèvements de cellules foetales, la confirmation de diagnostic de maladies génétiques comme la maladie de Huntington, la recherche de paternité, l'identification de criminels dans le cas de viol ou de meurtre, l'étude des populations de cellules cancéreuses dans les tumeurs, l'étude des RFLP (Restriction Fragment Length Polymorphism) lors des croisements génétiques.

**Revendications**

1. Procédé de coloration d'une séquence d'acide nucléique fixée sur un support solide, dans lequel on utilise un système enzymatique propre à se fixer sur cette séquence pour la détecter par un marquage de type non radioactif, et on fait réagir ce système enzymatique sur un substrat chromogénique pour former un produit coloré qui précipite in situ, ce qui permet de révéler la séquence détectée, caractérisé en ce que l'on utilise une solution de révélation comprenant :
– un composé organique soufré, possédant une liaison avec un atome de soufre, formant substrat pour le système enzymatique, et propre à être hydrolysé par le système enzymatique pour former un composé organique à fonction thiol; et
– un composé de métal, soluble et stable en solution aqueuse, propre à réagir avec le composé organique à fonction thiol pour former un complexe métal-composé soufré, qui présente une coloration spécifique dudit complexe, qui précipite in situ et qui peut être ensuite décoloré.

2. Procédé selon la revendication 1, caractérisé en ce que le métal dudit composé de métal est choisi parmi l'or, le mercure, l'argent, le platine et le plomb.

3. Procédé selon l'une des revendications 1 et 2, caractérisé en ce que ledit composé de métal est un composé de l'or propre à réagir avec le composé organique à fonction thiol pour former un complexe or-composé soufré de coloration jaune.

4. Procédé selon l'une des revendications 1 à 3, caractérisé en ce que l'on fait ensuite réagir le complexe métal-composé soufré avec un composé renforçateur propre à accentuer la coloration dudit complexe.

5. Procédé selon l'une des revendications 1 à 4, caractérisé en ce que l'on traite le complexe métal-composé soufré par une solution de décoloration.

6. Procédé selon l'une des revendications 1 à 5, caractérisé en ce que le système enzymatique comprend une enzyme à activité phosphatasique, par exemple la phosphatase alcaline, et en ce que la solution de révélation comprend un substrat phosphatasique, par exemple la cystéamine-S-phosphate, en tant que composé organique soufré ainsi qu'un composé organique du métal ou un sel du métal considéré.

7. Procédé selon la revendication 6, caractérisé en ce que le composé de métal est un composé de l'or et en ce que le système enzymatique comprend de la phosphatase alcaline et en ce que la solution de révélation comprend de la cystéamine-S-phosphate en tant que substrat et de l'aurothioglucose en tant que composé de l'or.

8. Procédé selon la revendication 7, caractérisé en ce que la solution de révélation comprend :

*NADPH = Nicotinamide Adénine Dinucléotide Phosphate, forme réduite

– du Tris-acétate 50 mM pH 9,5,
– de l'acétate de magnésium 10 mM,
– de la cystéamine-S-phosphate à 5 mM,
– de l'aurothioglucose 3 mM.

9. Procédé selon l'une des revendications 1 à 5, caractérisé en ce que le système enzymatique comprend l'enzyme glutathion réductase et en ce que la solution de révélation comprend un substrat de la glutathion réductase et un composé organique du métal ou un sel du métal considéré.

10. Procédé selon la revendication 9, caractérisé en ce que le composé de métal est un composé de l'or et en ce que la solution de révélation comprend :
– du Tris-HCl 50 mM à pH 7,
– du NADPH 5 mM,
– du Glutathion sous forme oxydée (sels de sodium) 5 mM,
– de l'HAuCl$_4$ ou de l'aurothioglucose 5 mM.

11. Procédé selon la revendication 4 , caractérisé en ce que le composé renforçateur est un sel d'argent, par exemple du nitrate d'argent, propre à réagir sur le complexe métal-composé soufré pour donner une coloration brun-noir.

12. Procédé selon la revendication 5, caractérisé en ce que la solution de décoloration comprend du thiosulfate de sodium et du thiosulfate d'ammonium à forte molarité en milieu acide.

13. Procédé selon la revendication 12, caractérisé en ce que, après traitement par la solution de décoloration, on traite le support par un mélange d'urée et de SDS à chaud pour éliminer toute trace de protéines et d'acides nucléiques, autres que la séquence d'acides nucléiques restant fixée sur le support solide.

14. Ensemble de réactifs pour la coloration d'une séquence d'acide nucléique fixée sur un support solide, en utilisant un système enzymatique propre à se fixer sur cette séquence pour la détecter par un marquage de type non radioactif, caractérisé en ce qu'il comprend :
– un flacon d'un composé organique soufré, possédant une liaison avec un atome de soufre, formant substrat pour le système enzymatique, et propre à être hydrolysé par ce système enzymatique pour former un composé organique à fonction thiol,
– un flacon d'un composé de métal, soluble et stable en solution aqueuse, propre à réagir avec le composé organique à fonction thiol pour former un complexe métal-composé soufré, de coloration spécifique dudit complexe et précipitant in situ,
– un flacon d'une solution tampon propre à être mélangé au substrat et au composé de métal pour former une solution de révélation, et
– un flacon d'une solution de décoloration.

15. Ensemble de réactifs selon la revendication 14, caractérisé en ce qu'il comprend en outre :

– un flacon d'une solution d'un composé renforçateur de coloration; et
– un flacon d'une solution d'urée et de SDS.

**Patentansprüche**

1. Verfahren zur Färbung einer Folge von auf einem festen Träger fixierten Nukleinsäuren, bei welchem ein Enzymsystem eingesetzt wird, der für den Nachweis dieser Folge mittels einer nichtradioaktiven Markierung an dieser Folge anlagerbar ist, und bei welchem man dieses Enzymsystem auf einem chromogenen Substrat reagieren läßt, um ein sich in situ niederschlagendes gefärbtes Produkt zu erhalten, welches die Entwicklung der nachgewiesenen Folge gestattet, dadurch gekennzeichnet, daß eine Entwicklungslösung eingesetzt wird, welche folgende Bestandteile enthält:
– eine schwefelhaltige organische Verbindung, die eine Bindung mit einem Schwefelatom besitzt, die ein Substrat für das Enzymsystem bildet und zur Bildung einer organischen Verbindung mit Thiolwirkung durch das Enzymsystem hydrolysierbar ist; und
– eine lösliche und in wäßriger Lösung stabile Metallverbindung, die zur Bildung eines komplexes aus Metall und schwefelhaltiger Verbindung mit der organischen Verbindung mit Thiolwirkung umsetzbar ist, wobei der Komplex eine spezifische Färbung aufweist, sich in situ niederschlägt und anschließend entfärbbar ist.

2. Verfahren nach Anspruch 1, dadurch gekennzeichnet, daß das Metall der Metallverbindung aus der Gruppe gewählt wird, die Gold, Quecksilber, Silber, Platin und Blei umfaßt.

3. Verfahren nach einem der Ansprüche 1 und 2, dadurch gekennzeichnet, daß die Metallverbindung eine Goldverbindung ist, welche zur Bildung eines gelb gefärbten Komplexes aus Gold und schwefelhaltiger Verbindung mit der organischen Verbindung mit Thiolwirkung umsetzbar ist.

4. Verfahren nach einem der Ansprüche 1 bis 3, dadurch gekennzeichnet, daß man anschließend den komplex aus Metall und schwefelhaltiger Verbindung mit einer Verstärkerverbindung reagieren läßt, mit welcher sich die Färbung des komplexes deutlicher hervorheben läßt.

5. Verfahren nach einem der Ansprüche 1 bis 4, dadurch gekennzeichnet, daß der komplex aus Metall und schwefelhaltiger Verbindung mit einer Entfärbelösung behandelt wird.

6. Verfahren nach einem der Ansprüche 1 bis 5, dadurch gekennzeichnet, daß das Enzymsystem ein Enzym mit Phosphatase- aktivität enthält, beispielsweise eine alkalische Phosphatase, und daß die Entwicklungslösung als schwefelhaltige organische Verbindung ein phosphatasehaltiges Substrat, beispielsweise Cysteamin-S-phosphat, sowie eine organische Lösung Des jeweiligen Metalls bzw. eines Salzes dieses Metalls enthält.

7. Verfahren nach Anspruch 6, dadurch gekennzeichnet, daß die Metallverbindung eine Goldverbindung ist, und daß das Enzymsystem alkalische Phosphatase enthält, und daß ferner die Entwicklungslösung als Substrat Cysteamin-S-phosphat und als Goldverbindung Aurothioglukose enthält.

8. Verfahren nach Anspruch 7, dadurch gekennzeichnet, daß die Entwicklungslösung folgende Bestandteile enthält:
 – Trisazetat, 50 mM, pH-Wert 0,5,
 – Magnesiumazetat, 10 mM
 – Cysteamin-S-phosphat zu 5 mM,
 – Aurothioglukose, 3 mM.

9. Verfahren nach einem der Ansprüche 1 bis 5, dadurch gekennzeichnet, daß das Enzymsystem das Enzym Glutathion-Reduktase enthält, und daß die Entwicklungslösung ein Glutathion-Reduktase-Substrat und eine organische Verbindung des jeweiligen Metalls oder eines Salzes dieses Metalls enthält.

10. Verfahren nach Anspruch 9, dadurch gekennzeichnet, daß die Metallverbindung eine Goldverbindung ist, und daß die Entwicklungslösung folgende Bestandteile enthält:
 – Tris-HCl, 50 mM, mit einem pH-Wert von 7,
 – NADPH, 5 mM,
 – Glutathion in oxidierter Form (Natriumsalze), 5 mM,
 – HAuCl$_4$ bzw. Aurothioglukose, 5 mM.

11. Verfahren nach Anspruch 4, dadurch gekennzeichnet, daß die Verstärkerverbindung ein Silbersalz, beispielsweise Silbernitrat, ist, das auf dem Komplex aus Metall und schwefelhaltiger Verbindung unter Herbeiführung einer braun-schwarzen Färbung umsetzbar ist.

12. Verfahren nach Anspruch 5, dadurch gekennzeichnet, daß die Entfärbelösung Natriumthiosulfat und Ammoniumthiosulfat mit hoher Molarität in saurem Milieu enthält.

13. Verfahren nach Anspruch 12, dadurch gekennzeichnet, daß nach der Behandlung mit der Entfärbelösung der Träger mit einer Mischung aus Carbamid und SDS im warmen Zustand behandelt wird, um alle Spuren von Proteinen und Nukleinsäuren außer der Folge der Nukleinsäuren, die auf dem festen Träger fixiert bleiben, zu entfernen.

14. Gesamtkomplex von Reagenzien zur Färbung einer auf einem festen Träger fixierten Folge von Nukleinsäuren unter Verwendung eines zum Nachweis dieser Folge mittels einer nichtradioaktiven Markierung an dieser Folge anlagerbaren Enzymsystems, dadurch gekennzeichnet, daß der Gesamtkomplex folgendes umfaßt:
 – einen Kolben mit einer schwefelhaltigen organischen Verbindung, die eine Bindung mit einem Schwefelatom besitzt, die ein Substrat für das Enzymsystem bildet und zur Bildung einer organischen Verbindung mit Thiolwirkung durch das Enzymsystem hydrolysierbar ist; und
 – einen Kolben mit einer löslichen und in wäßriger Lösung stabilen Metallverbindung, die zur Bildung eines komplexes aus Metall und schwefelhaltiger Verbindung mit der organischen Verbindung mit Thiolwirkung umsetzbar ist, wobei der Komplex eine spezifische Färbung aufweist und sich in situ niederschlägt,
 – einen Kolben mit einer Pufferlösung, welche zur Bildung einer Entwicklungslösung mit dem Substrat und der Metallverbindung vermischbar ist, und
 – einen Kolben mit einer Entfärbelösung.

15. Gesamtkomplex von Reagenzien nach Anspruch 14, dadurch gekennzeichnet, daß er außerdem umfaßt:
 – einen Kolben mit einer Farbverstärkerlösung, und
 – einen Kolben mit einer Lösung von Carbamid und SDS.

## Claims

1. Process for staining a nucleic acid sequence fixed to a solid support, in which an enzyme system is used which is capable of being fixed to this sequence in order to detect it by way of non-radioactive labelling, this enzyme system being made to react with a chromogenic substrate in order to form a stained product which precipitates in situ, which allows the revealing of the detected sequence, characterised in that a disclosing solution is used, comprising:
 – an organic sulphur-containing compound, having a bond with one sulphur atom, forming a substrate for the enzyme system, and capable of being hydrolysed by the enzyme system in order to form an organic compound with a thiol function; and
 – a metal compound, which is soluble and stable in aqueous solution, capable of reacting with the organic compound with a thiol function to form a complex of metal and sulphur-containing compound which shows a specific colouration of said complex, which precipitates in situ and which can subsequently be bleached.

2. Process according to claim 1, characterised in that the metal of said metal compound is chosen from gold, mercury, silver, platinum and lead.

3. Process according to either of claims 1 and 2, characterised in that said metal compound is a gold compound capable of reacting with the organic compound with a thiol function to form a complex of gold and sulphur-containing compound of a yellow colouration.

4. Process according to one of claims 1 to 3, characterised in that the complex of metal and sulphur-containing compound is then made to react with a booster compound capable of accentuating the colouration of said complex.

5. Process according to one of claims 1 to 4, characterised in that the complex of metal and sulphur-containing compound is reated with a bleaching solution.

6. Process according to one of claims 1 to 5, characterised in that the enzyme system comprises an enzyme with phosphatase action, alkaline phosphatase for example, and in that the disclosing solution comprises a phosphatase substrate, e.g. cysteamine-S-phosphate, as an organic sulphur-containing compound, as well as an organic compound of the metal or a salt of the metal in question.

7. Process according to claim 6, characterised in that the metal compound is a gold compound, and in that the enzyme system comprises alkaline phosphatase, and in that the disclosing solution comprises cysteamine-S-phosphate as a substrate and gold thioglucose as a gold compound.

8. Process according to claim 7, characterised in that the disclosing solution comprises:
   – tris-acetate, 50 mM pH 9.5,
   – magnesium acetate, 10 mM,
   – cysteamine-S-phosphate, 5mM,
   – gold thioglucose, 3mM.

9. Process according to one of claims 1 to 5, characterised in that the enzyme system comprises the enzyme glutathione reductase, and in that the disclosing solution comprises a substrate of glutathione reductase and an organic compound of the metal or of a salt of the metal in question.

10. Process according to claim 9, characterised in that the metal compound is a gold compound, and in that the disclosing solution comprises:
   – tris-HCl, 50mM at pH7,
   – NADPH, 5mM,
   – glutathione in an oxidised form (sodium salt), 5mM,
   – $HAuCl_4$ or gold thioglucose, 5mM.

11. Process according to claim 4, characterised in that the booster compound is a silver salt, e.g. silver nitrate, capable of reacting with the complex of metal and sulphur-containing compound to give a brown-black colouration.

12. Process according to claim 5, characterised in that the bleaching solution comprises sodium thiosulphate and ammonium thiosulphate in high molarity in an acid medium.

13. Process according to claim 12, characterised in that, after treatment by the bleaching solution, the support is hot-treated by a mixture of urea and SDS to eliminate any trace of proteins and nucleic acids, other than the sequence of nucleic acids remaining fixed to the solid support.

14. Kit for staining a nucleic acid sequence fixed to a solid support, using an enzyme system capable of being fixed to this sequence in order to detect it by non-radioactive labelling, characterised in that it comprises:
   – a bottle of an organic sulphur-containing compound, having a bond with one sulphur atom, forming a substrate for the enzyme system, and capable of being hydrolysed by the enzyme system to form an organic compound with a thiol function,
   – a bottle of a metal compound, which is soluble and stable in aqueous solution, capable of reacting with the organic compound with a thiol function to form a complex of metal and sulphur-containing compound, of a specific colouration of said complex and precipitating in situ,
   – a bottle of a buffer solution capable of being mixed with the substrate and with the metal compound to form a disclosing solution, and
   – a bottle of a bleaching solution.

15. Kit according to claim 14, characterised in that it additionally comprises:
   – a bottle of a stain booster compound in solution; and
   – a bottle of a solution of urea and SDS.